# EUROPEAN PATENT SPECIFICATION

(11) **EP 3 034 120 B1**
(45) Date of publication and mention of the grant of the patent: **20.06.2018**
(21) Application number: 15199832.5
(22) Date of filing: 12.12.2013
(51) Int. Cl.: A61M 16/08, A61M 16/14, A61M 39/10, A61M 16/00, A61M 39/08, A61M 16/06

(54) **ROTARY FLUID COUPLER**
DREHFLUIDKOPPLER
COUPLEUR DE FLUIDE ROTATIF

(30) Priority: 17.12.2012 US 201261738091 P
(43) Date of publication of application: 22.06.2016
(62) Divisional of application: 13831904.1
(73) Proprietor: Koninklijke Philips N.V., 5656 AE Eindhoven (NL)
(72) Inventor: BAFILE, Anthony Jon, 5656 AE Eindhoven (NL); SHELLY, Benjamin Irwin, 5656 AE Eindhoven (NL)
(74) Representative: Schudelaro, Antonius Adrianus Petrus

(56) References cited:
- US-A- 5 617 847
- US-A1- 2008 011 368
- US-A1- 2008 142 019
- US-A1- 2009 133 697

## Description

### BACKGROUND OF THE INVENTION

### 1. Field of the Invention

The present invention pertains to fluid coupling devices, and, in particular, to a rotary fluid coupling device that has multiple, separate channels and that allows for portions of the coupling device to rotate relative to one another while maintaining the integrity of the separate channels. Such a rotary fluid coupling device would, for example, be useful in a medical application, such as a pressure support system for treating sleep apnea, wherein breathing gas would delivered to the patient through one channel of the coupling device and the other channel or channels would be used for another purpose, such as pressure monitoring or the delivery of supplemental oxygen to the patient.

### 2. Description of the Related Art

There are numerous situations where it is necessary or desirable to deliver a flow of breathing gas non-invasively to the airway of a patient, i.e., without intubating the patient or surgically inserting a tracheal tube into the patient's esophagus. For example, it is known to ventilate a patient using a technique known as non-invasive ventilation. It is also known to deliver positive airway pressure (PAP) therapy to treat certain medical disorders, the most notable of which is OSA. Known PAP therapies include continuous positive airway pressure (CPAP), wherein a constant positive pressure is provided to the airway of the patient in order to splint open the patient's airway, and variable airway pressure, wherein the pressure provided to the airway of the patient is varied with the patient's respiratory cycle. Such therapies are typically provided to the patient at night while the patient is sleeping.

Non-invasive ventilation and pressure support therapies as just described involve the placement of a patient interface device including a mask component having a soft, flexible sealing cushion on the face of a patient. The mask component may be, without limitation, a nasal mask that covers the patient's nose, a nasal/oral mask that covers the patient's nose and mouth, or a full face mask that covers the patient's face. Such patient interface devices may also employ other patient contacting components, such as forehead supports, cheek pads and chin pads. The patient interface device is connected to a gas delivery tube or conduit and interfaces the ventilator or pressure support device with the airway of the patient, so that a flow of breathing gas can be delivered from the pressure/flow generating device to the airway of the patient. It is known to maintain such devices on the face of a wearer by a headgear having one or more straps adapted to fit over/around the patient's head.

In addition, it is very common for the gas delivery tube or conduit to be connected to the patient interface device with a rotary coupler (also commonly known as a swivel connector) to enhance comfort and prevent twisting of the gas delivery tube or conduit. Also, it is not uncommon to require pressure feedback from the patient. An accurate way of measuring patient pressure is by placing a pressure pickoff port in the mask of the patient interface device and utilizing a pressure tube to relay the pressure feedback to a sensor in the flow generating device. However, a traditional delivery conduit (e.g., a traditional hose) connected to a traditional rotary coupler or swivel port can experience undesirable tangling/twisting due to the pressure tube, which often limits the ability of the delivery conduit to rotate.

US 2009/0133697 A1 discloses a connector system for an apparatus that delivers breathable gas to a patient. The connector system includes an air delivery conduit including an auxiliary conduit. The air delivery conduit and the auxiliary conduit have a first end and a second end. A connector is provided to the at least one of the first and the second ends. The connector includes an inner tubular wall and an outer tubular wall surrounding the inner tubular wall and being concentric with the inner tubular wall. The inner and outer tubular walls define first and second passages that are isolated from one another. One of the first and second passages is configured to communicate with the air delivery conduit and the other of the first and the second passages is configured to communicate with the auxiliary conduit.

US 2008/0011368 A1 discloses an inline swivel connection for multi-lumen tubing.

Further exemplary couplings and connections for tubings of respiratory interface devices are known from US 2008/0142019 A1, US 5,617,847 A, and from US 5,284,160 A.

### SUMMARY OF THE INVENTION

The invention is defined in independent claim 1. In one embodiment, a system for delivering a flow of breathing gas to a patient is provided that includes a main delivery conduit coupled to a source of the flow of the breathing gas, and a rotary coupling device coupled to the main delivery conduit. The rotary coupling device has a first member and a second member, the first member defining a first channel through the first member and a second channel through the first member, the second member defining a third channel through the second member and a fourth channel through the second member. The first channel is in communication with the third channel to define a main channel having a main path through the rotary coupling device that is in communication with the main delivery conduit, and the second channel is in communication with the fourth channel to define a secondary channel separate from the main channel and having a secondary path through the rotary coupling device. The first member and the second member are structured to freely rotate relative to one another for unlimited, 360° rotation about a longitudinal axis of the rotary coupling device in a manner that separately maintains the integrity of the main path and the secondary path
wherein the first member comprises a first cylindrical portion and a second, wider cylindrical portion coupled to and extending from the first cylindrical portion, the first cylindrical portion including a first inner cylindrical wall and the second cylindrical portion including a first outer cylindrical wall and a second inner cylindrical wall, wherein the first member further comprises a first port that provides access to a space between the first outer cylindrical wall and the second inner cylindrical wall, wherein the first and second inner cylindrical walls define the first channel and the first port comprises part of the second channel; and
wherein the second member comprises a third cylindrical portion and a fourth, narrower cylindrical portion coupled to and extending from the third cylindrical portion, the third cylindrical portion including a second outer cylindrical wall and a third inner cylindrical wall, wherein the second member further comprises a second port that provides access to a space between the second outer cylindrical wall and the third inner cylindrical wall, wherein the third inner cylindrical wall and the fourth cylindrical portion together define the third channel and the second port comprises part of the fourth channel,
wherein a chamber is formed between the second cylindrical portion of the first coupling member and the third cylindrical portion of the second coupling member, wherein the chamber, the first port and the second port together form the secondary channel.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1 is a schematic side view of a pressure support system adapted to provide a regimen of respiratory therapy to a patient according to one exemplary embodiment.
FIG. 2 is an isometric view of a rotary coupling device according to a non-claimed example that may be employed in the pressure support system of FIG. 1.
FIG. 3 is an exploded view of the rotary coupling device shown in FIG. 2.
FIG. 4 is a cross-sectional isometric view of the rotary coupling device shown in FIG. 2.
FIG. 5 is an isometric view of a rotary coupling device according to the invention that may be employed in the pressure support system of FIG. 1.
FIG. 6 is an exploded view of the rotary coupling device shown in FIG. 5.
FIG. 7 is a cross-sectional isometric view of the rotary coupling device shown in FIG. 5.
FIGS. 8-10 are schematic side views of pressure support systems adapted to provide a regimen of respiratory therapy to a patient according to various alternative exemplary embodiments.
FIG. 11 is a schematic view of a tracheal intubation apparatus according to another alternative exemplary embodiment.
FIG. 12 is an isometric view of a rotary coupling device according to a non-claimed example.
FIG. 13 is a cross-sectional isometric view of the rotary coupling device shown in FIG. 12.
FIG. 14 is an isometric view of a first coupling member of the rotary coupling device shown in FIG. 6.
FIG. 15 is an isometric view of a second coupling member of the rotary coupling device shown in FIG. 6.

### DETAILED DESCRIPTION OF EXEMPLARY EMBODIMENTS

As used herein, the singular form of "a," "an," and "the" include plural references unless the context clearly dictates otherwise. As used herein, the statement that two or more parts or components are "coupled" shall mean that the parts are joined or operate together either directly or indirectly, *i.e.,* through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (*i.e.*, a plurality).

As used herein, a "substantially fluid tight seal" means that two surfaces sealingly engage each other in a manner that substantially limits passage of a fluid between the two surfaces (e.g., no more than 5% passage).

As used herein, the term "sealingly" or "sealed" in the context of an engagement, attachment or coupling means that two parts are coupled to one another with a substantially fluid tight seal.

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

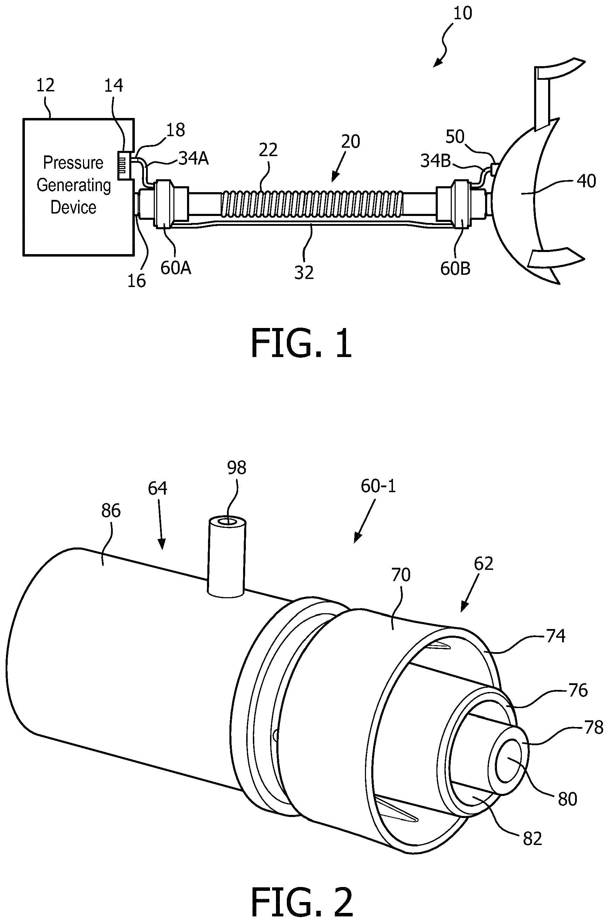

A pressure support system 10 adapted to provide a regimen of respiratory therapy to a patient according to one exemplary embodiment is generally shown in FIG. 1. Pressure support system 10 includes a pressure generating device 12, a patient interface device 40, and a patient circuit 20. As described in greater detail herein, patient circuit 20 employs a number of rotary coupling devices (labeled 60) that each contain a main channel for communication/delivery of the therapy breathing gas generated by pressure generating device 12 (e.g., therapy air) and at least one separate secondary channel for another purpose, which, in the exemplary embodiment of FIG. 1, is pressure feedback, but which may also be for other purposes, such as, without limitation, communication/delivery of supplemental oxygen. As also described in greater detail herein, each rotary coupling device provides for unlimited, 360 degree rotation while maintaining the integrity of the separate channels. As will be appreciated, this reduces the chances that undesirable hose/tube twisting will occur. In addition, in the exemplary embodiment, for patient comfort, rotational friction within the each rotary coupling device will be minimal.

Furthermore, although pressure support system 10 is discussed as including pressure generating device 12, patient interface 40 and patient circuit 20, it is contemplated that other systems may employ the concepts of the rotary coupling device described herein. Examples of such system are discussed elsewhere herein.

Referring again to FIG. 1, pressure generating device 12 is structured to generate a flow of breathing gas and may include, without limitation, ventilators, constant pressure support devices (such as a continuous positive airway pressure device, or CPAP device), variable pressure devices (e.g., BiPAP®, Bi-Flex®, or C-Flex™ devices manufactured and distributed by Philips Respironics of Murrysville, Pennsylvania), and auto-titration pressure support devices. Pressure generating device 12 includes a pressure sensor 14, a delivery conduit coupling 16 coupled to the source of gas within pressure generating device 12, and a pressure conduit coupling 18 coupled to pressure sensor 14 of pressure generating device 12 and structured to receive pressure feedback as described elsewhere herein.

In the exemplary embodiment, patient interface device 40 comprises a nasal/oral mask that is structured to be placed over the nasal and oral orifices of a patient. It is to be understood, however, that patient interface device 40 can include alternative types of mask/sealing portions, such as, without limitation, a nasal mask, a pillow style nasal cushion, a cradle style nasal cushion, a full face mask, any other device that provides a suitable gas flow communicating function.

Patient circuit 20 is structured to both (i) communicate the flow of breathing gas from pressure generating device 12 to patient interface device 40, and (ii) provide pressure feedback from patient interface device 40 to pressure generating device 12 (i.e., to pressure sensor 14). In the exemplary embodiment, patient circuit 20 includes a main delivery conduit 22, a main pressure feedback conduit 32, first and second rotary coupling devices 60A and 60B, described in greater detail herein, and first and second secondary pressure feedback conduits 34A, 34B. As seen in FIG. 1, the first end of delivery conduit 22 is coupled to the main channel of rotary coupling device 60A at the first end of rotary coupling device 60A, with the main channel of rotary coupling device 60A also being connected to pressure generating device 12 through the connection of the second end of rotary coupling device 60A to delivery conduit coupling 16. As also seen in FIG. 1, the second end of delivery conduit 22 is coupled to the main channel of rotary coupling device 60B at the first end of rotary coupling device 60B, with the main channel of rotary coupling device 60B also being connected to patient interface device 40 through the connection of the second end of rotary coupling device 60B to a fluid inlet of a mask portion of patient interface device 40.

Furthermore, secondary pressure feedback conduit 34A provides a connection between the secondary channel of rotary coupling device 60A and pressure conduit coupling 18 of pressure generating device 12 (and thus an operative connection to pressure sensor 14), and secondary pressure feedback conduit 34B provides a connection between the secondary channel of rotary coupling device 60B and a port 50 of the mask portion of patient interface device 40. Port 50 is configured to allow a measurement of pressure at patient interface device 40 to be made. In addition, main pressure feedback conduit 32 provides a connection between the secondary channel of rotary coupling device 60A and the secondary channel of rotary coupling device 60B.

Thus, in the configuration just described, patient circuit 20 provides both (i) a main path or channel for communicating the flow of breathing gas from pressure generating device 12 to patient interface device 40 that utilizes main delivery conduit 22 and the main channels of rotary coupling device 60A and 60B, and (ii) a pressure feedback path or channel from patient interface device 40 to pressure generating device 12 that utilizes pressure feedback conduit 32 and the secondary channels of rotary coupling device 60A and 60B. In addition, due to the rotating nature of rotary coupling devices 60A and 60B, such paths or channels are able to be established and maintained with a reduced risk of conduit/hose twisting within pressure support system 10.

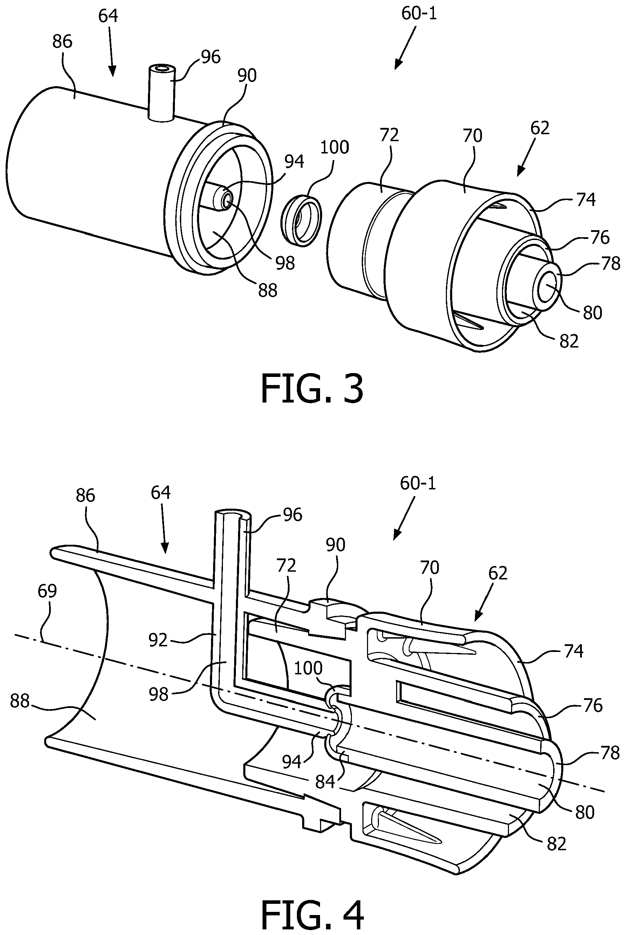

FIG. 2 is an isometric view, FIG. 3 is an exploded view, and FIG. 4 is a cross-sectional isometric view of a non-claimed example of rotary coupling device 60 (labeled 60-1) that may be used for either or both of rotary coupling device 60A and rotary coupling device 60B shown in FIG. 1. Rotary coupling device 60-1 includes a first coupling member 62 and a second coupling member 64 that are structured to be coupled to one another (as described in greater detail herein) in a manner that defines multiple separate channels within rotary coupling device 60-2 and wherein first coupling member 62 and second coupling member 64 are able to rotate relative to one another about longitudinal axis 69 shown in FIG. 4. In the illustrated example, a simple annular snap connection is employed to rotatably couple first coupling member 62 to second coupling member 64.

As seen in FIGS. 2-4, first coupling member 62 includes a first cylindrical portion 70 and a second, narrower cylindrical portion 72 coupled to and extending from first cylindrical portion 70. In the illustrated example, first cylindrical portion 70 comprises a three-walled structure including an outer cylindrical wall 74 (which functions as an outer sheath member), a middle cylindrical wall 76 and an inner cylindrical wall 78 (in an alternative example, outer cylindrical wall 74 may be omitted, as it is not necessary to create the fluid passages discussed below). Inner cylindrical wall 78 defines a first passage 80 thorough first coupling member 62, and the space between middle cylindrical wall 76 and inner cylindrical wall 78 defines a second passage 82 thorough first coupling member 62. The purpose of each of the first passage 80 and the second passage 82 is described below. Furthermore, while in the illustrated example inner cylindrical wall 78 is straight, it will be understood that it may take on other shapes (e.g., L-shaped and exiting outer cylindrical wall 74 and middle cylindrical wall 76 similar to internal tube member 92 described below) are also possible for defining first passage 80. In addition, the outer surface of middle cylindrical wall 76 provides a surface to which delivery conduit 22 may be sealingly attached. In addition, as seen in FIG. 4, the end of inner cylindrical wall 78 closest to cylindered portion 72 includes a flange 84, the purpose of which is described elsewhere herein.

Second coupling member 64 includes a main outer wall 86 that defines a main chamber or passage 88. Second coupling member 64 also includes a first end 90 that is structured to sealingly and rotatably engage second cylindrical portion 72 of first coupling member 62. Second coupling member 64 also further includes an internal tube member 92 having a first end 94 provided at first end 90 of second coupling member 64, and a second end 96 that extends out of main outer wall 86. In the illustrated example, tube member 92 is L-shaped, but it will be understood that other shapes (e.g., straight) are also possible. Tube member 92 defines an internal passage 98 of second coupling member 64.

In the exemplary example, rotary coupling device 60-1 also includes a rotary seal member 100. As shown in FIG. 4, rotary seal member 100 is structured to provide a substantially fluid tight seal between flange 84 of first coupling member 62 and first end 94 of tube member 92 in a manner wherein flange 84 and first end 94 of tube member 92 are able to rotate relative to one another with minimal friction. In the exemplary example, rotary seal member 100 is a flexible, bellows style seal or gasket member made of any suitable material such as rubber or silicone.

In the exemplary example, rotary coupling device 60-1 is assembled in the following manner. First, a proximal end of rotary seal member 100 is attached to flange 84. Then, second cylindrical portion 72 of first coupling member 62 is inserted into first end 90 of second coupling member 64 to create the rotatable connection between the two components. When this is done, care is taken to ensure that first end 94 of tube member 92 is received in the distal end of rotary seal member 100.

In an alternative example, rotary seal member 100 may be omitted, in which case flange 84 and first end 94 of tube member 92 are interference or snap fit to one another (to provide a substantially fluid tight seal) in a manner that allows for rotation relative to one another with minimal friction.

When assembled as just described, rotary coupling device 60-1 will include the two separate channels described elsewhere herein. In particular, second passage 82 of first coupling member 62 and main chamber or passage 88 of second coupling member 64 will together form the main channel of rotary coupling device 60-1 (e.g., for communicating breathing gas as shown in FIG. 1), and first passage 80 of first coupling member 62 and internal passage 98 of second coupling member 64 will together form the secondary channel of rotary coupling device 60-1 (e.g., for providing pressure feedback as shown in FIG. 1).

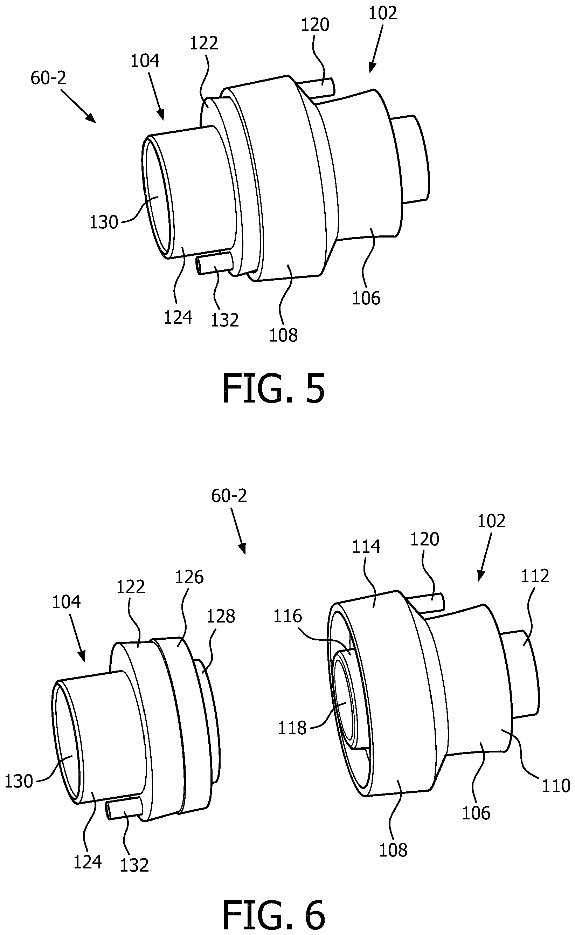

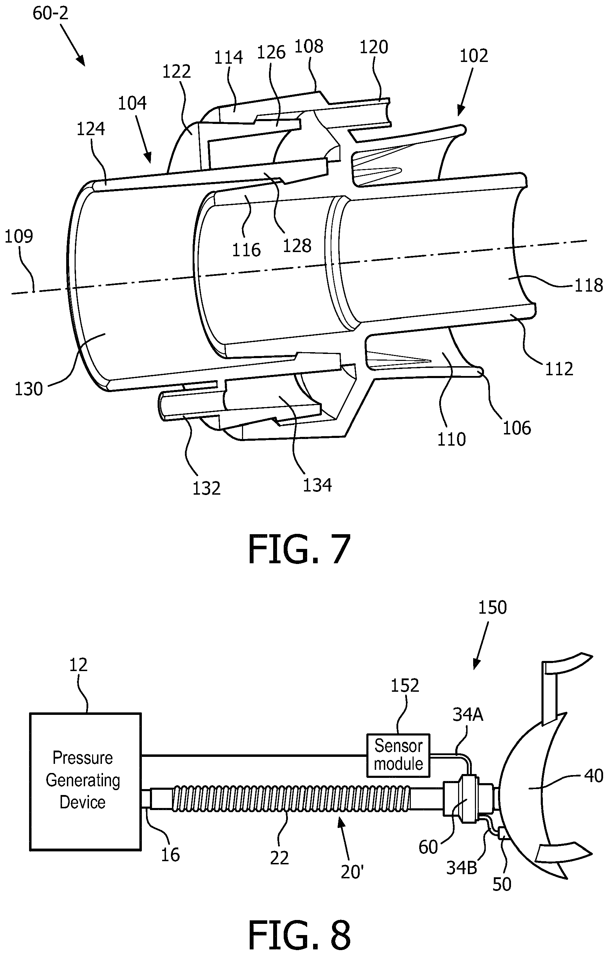

FIG. 5 is an isometric view, FIG. 6 is an exploded view, and FIG. 7 is a cross-sectional view of a particular exemplary embodiment of rotary coupling device 60 (labeled 60-2) that may be used for either or both of rotary coupling device 60A and rotary coupling device 60B shown in FIG. 1. Rotary coupling device 60-2 includes a first coupling member 102 and a second coupling member 104 that are structured to be coupled to one another (as described in greater detail herein) in a manner that defines multiple separate channels within rotary coupling device 60-2 and wherein first coupling member 102 and second coupling member 104 are able to rotate relative to one another about longitudinal axis 109 shown in FIG. 7. In the illustrated embodiment, a simple annular snap connection is employed to rotatably couple first coupling member 102 to second coupling member 104.

As seen in FIGS. 5-7, first coupling member 102 includes a first cylindrical portion 106 and a second, wider cylindrical portion 108 coupled to and extending from first cylindrical portion 106. First cylindrical portion 106 comprises a two-walled structure including an outer cylindrical wall 110 and an inner cylindrical wall 112. Similarly, second cylindrical portion 108 also comprises a two-walled structure including an outer cylindrical wall 114 and an inner cylindrical wall 116. Inner cylindrical walls 112 and 116 together define a main passage 118 through first coupling member 102. In addition, as seen FIGS. 5-7, first coupling member 102 includes a port 120 that provides access to the space between outer cylindrical wall 114 and inner cylindrical wall 116. The significance of this is described elsewhere herein. The outer surface of inner cylindrical wall 112 provides a surface to which delivery conduit 22 is sealingly attached.

Second coupling member 104 includes a first cylindrical portion 122 and a second, narrower cylindrical portion 124 coupled to and extending from first cylindrical portion 122. First cylindrical portion 122 comprises a two-walled structure including an outer cylindrical wall 126 and an inner cylindrical wall 128. Inner cylindrical wall 128 and second cylindrical portion 124 together define a main passage 130 thorough second coupling member 104. In addition, as seen FIGS. 5-7, second coupling member 104 includes a port 132 that provides access to the space between outer cylindrical wall 126 and inner cylindrical wall 128. The significance of this is described elsewhere herein.

In the exemplary embodiment, rotary coupling device 60-2 is assembled by inserting inner cylindrical wall 116 of first coupling member 102 into inner cylindrical wall 128 of second coupling member 104. At the same time, first cylindrical portion 122 of second coupling members 104 is received within the space between outer cylindrical wall 114 and an inner cylindrical wall 116 of first coupling member 102. When this is done, a chamber 134 (FIG. 7) will be formed between second cylindrical portion 108 of first coupling member 102 and first cylindrical portion 122 of second coupling member 104, with access to chamber 134 being provided by ports 120 and 132.

When assembled as just described, rotary coupling device 60-2 will include the two separate channels described elsewhere herein. In particular, main passage 118 of first coupling member 102 and main passage 130 of second coupling member 104 will together form the main channel of rotary coupling device 60-2 (e.g., for communicating breathing gas as shown in FIG. 1), and chamber 134 and ports 120 and 132 will together form the secondary channel of rotary coupling device 60-2 (e.g., for providing pressure feedback as shown in FIG. 1).

It is noted that in the rotary coupling device 60-2, first coupling member 102 and second coupling member 104 are not necessarily structured to be connected to one another with a substantially fluid tight seal. Thus, fluid chamber 134 may leak therefrom. This leak may be characterized and compensated for at various pressure settings on pressure generating device 12 of FIG. 1.

In the exemplary embodiments described herein, there two separate channels, a main channel and a secondary channel. It will be understood, however, that rotary coupling device 60 as described herein may include one or more additional secondary channels. Regardless of the number of channels, rotary coupling device 60 allows the elements coupled thereto, *e.g.* patient interface device 40 and delivery conduit 22, to swivel, or rotate, relative to each other while maintaining the integrity of the separate channels.

A pressure support system 150 adapted to provide a regimen of respiratory therapy to a patient according to an alternative exemplary embodiment is generally shown in FIG. 8. Pressure support system 150 is similar to pressure support system 10, and like components are labeled with like reference numerals. However, pressure support system 150 includes a modified patient circuit 20' that includes a single rotary coupling device 60 (e.g., 60-1, 60-2 or 60-3 (which is described below)). As seen in FIG. 8, the secondary channel of rotary coupling device 60 is used to provide pressure feedback to a sensor module 152 that includes a pressure sensor. Sensor module 152 is separate from pressure generating device 12, and, in the exemplary embodiment, is positioned close to patient interface device 40. Sensor module 152 is in wired or wireless communication with pressure generating device 12 in order to provide the pressure data measured by sensor module 152 to pressure generating device 12.

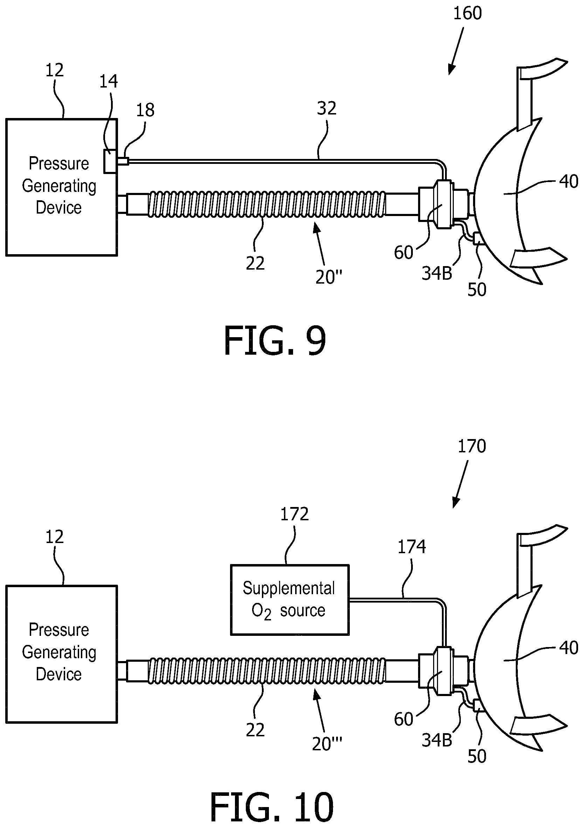

A pressure support system 160 adapted to provide a regimen of respiratory therapy to a patient according to another alternative exemplary embodiment is generally shown in FIG. 9. Pressure support system 160 is similar to pressure support systems 10 and 150, and like components are labeled with like reference numerals. However, pressure support system 160, like pressure support system 150, includes a modified patient circuit 20" that includes a single rotary coupling device 60 (e.g., 60-1, 60-2 or 60-3 (which is described below)), wherein, as seen in FIG. 9, the secondary channel of rotary coupling device 60 is used to provide pressure feedback to the pressure sensor 14 of pressure generating device 12 using a main pressure feedback conduit 32.

A pressure support system 170 adapted to provide a regimen of respiratory therapy to a patient according to yet another alternative exemplary embodiment is generally shown in FIG. 10. Pressure support system 170 is similar to pressure support systems 10, 150 and 160, and like components are labeled with like reference numerals. However, pressure support system 170 includes a modified patient circuit 20'" that includes a single rotary coupling device 60 (e.g., 60-1, 60-2, or 60-3 (which is described below)), wherein, as seen in FIG. 10, the secondary channel of rotary coupling device 60 is used enable a supplemental gas, such as, without limitation, oxygen, to be provided to patient interface device 40 from supplemental gas (e.g., oxygen) source 172 though secondary conduit 174. In one alternative embodiment, the supplemental gas may be CO2, which is used to treat Cheyne Stokes respiration.

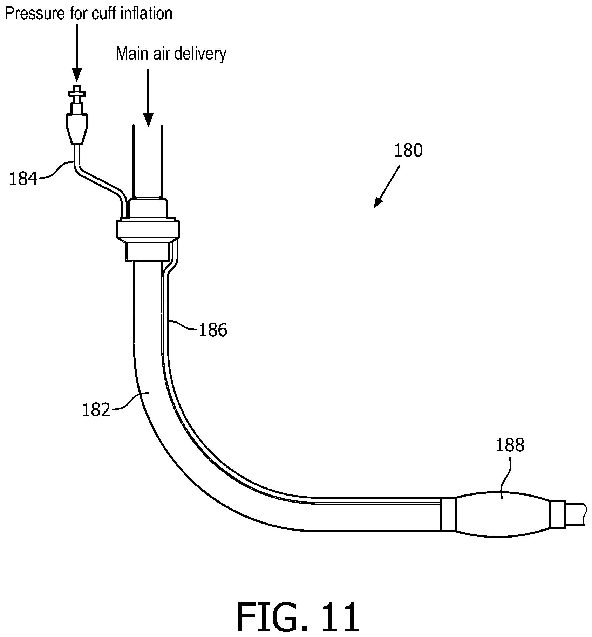

In still another alternative embodiment, a rotary coupling device 60 e.g., 60-1, 60-2, or 60-3 (which is described below)) maybe used to pass pressure for an inflatable cuff on tracheal intubation equipment. More specifically, referring to FIG. 11, a tracheal intubation apparatus 180 according to an exemplary embodiment is shown. Tracheal intubation apparatus 180 includes a rotary coupling device 60, a main therapy delivery tube/line 182, a first cuff inflation line 184, a second cuff inflation line 186 and an inflatable cuff 188 in fluid communication with second cuff inflation line 186. Main therapy delivery tube/line 182 is coupled to the main channel of rotary coupling device 60 to enable air to be delivered to the airway of the patient through the main channel of rotary coupling device 60 and main therapy delivery tube/line 182. In addition, as shown in FIG. 11, first cuff inflation line 184 and second cuff inflation line 186 are coupled to the secondary channel of rotary coupling device 60 to enable pressurized air generated at first cuff inflation line 184 to be delivered to second cuff inflation line 186 and inflatable cuff 188 through the secondary channel of rotary coupling device 60..

In yet another alternative embodiment, a rotary coupling device 60 e.g., 60-1, 60-2, or 60-3 (which is described below)) may be used to pass fluid through the secondary channel that is aerosolized at the mask or a separate mechanism. For example, the fluid may be water for humidification or a medication (such as albuterol) for nebulization.

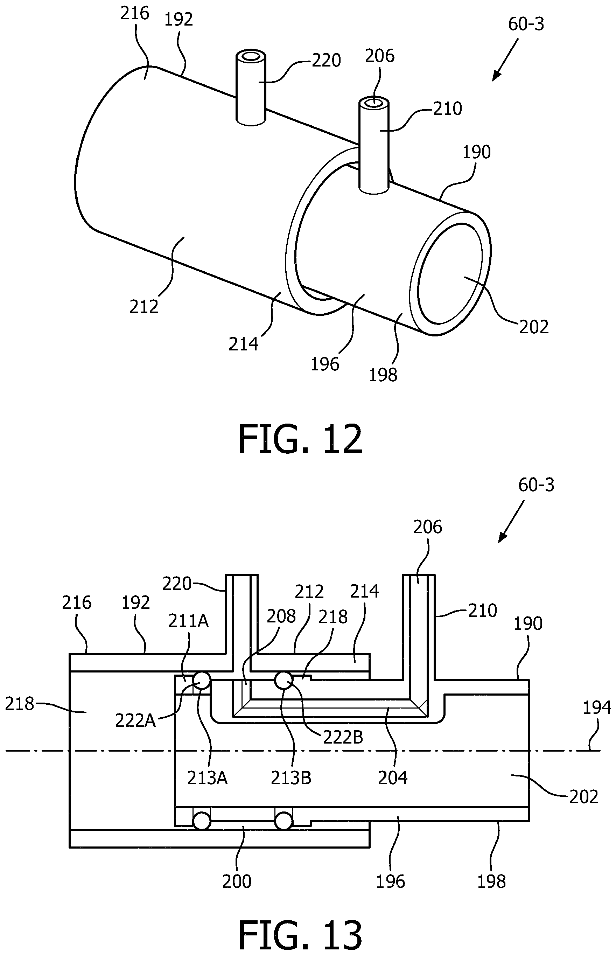

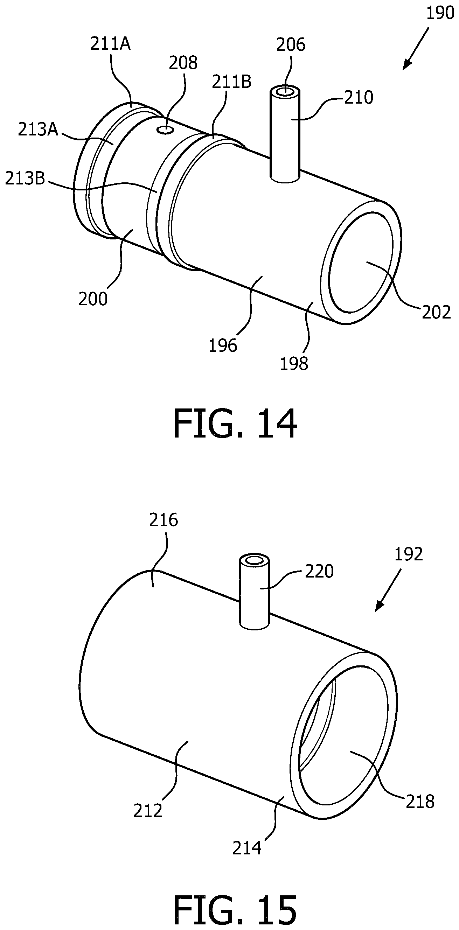

FIG. 12 is an isometric view and FIG. 13 is a cross-sectional view of a further non-claimed example of rotary coupling device 60 (labeled 60-3). Rotary coupling device 60-3 includes a first coupling member 190 and a second coupling member 192 that are structured to be coupled to one another in a manner that defines multiple separate channels within rotary coupling device 60-3 and wherein first coupling member 190 and second coupling member 192 are able to rotate relative to one another about longitudinal axis 194 shown in FIG. 13. FIG. 14 is an isometric view of first coupling member 190 and FIG. 15 is an isometric view of second coupling member 190.

As seen in FIGS. 13 and 14, first coupling member 190 includes a cylindrical body portion 196 having a first end 198 and a second end 200. Cylindrical body portion 196 defines a main passage 202 through first coupling member 190. Cylindrical body portion 196 also includes a secondary passage 204 having a first port 206 and a second port 208. First port 206 is provided on a tube member 210 that extends from the outer surface of first end 198 of first coupling member 190, and second port 208 is in the form of an aperture provided in second end 200 of first coupling member 190. In addition, second end 200 of first coupling member 190 includes ridge members 211A and 211B and grooves 213A and 213B on opposite sides of second port 208. The function of these elements is described below. The outer surface of first end 198 of first coupling member 190 provides a surface to which a delivery conduit may be sealingly attached.

As seen in FIGS. 13 and 15, second coupling member 192 includes a cylindrical body portion 212 having a first end 214 and a second end 216. Cylindrical body portion 212 defines a main passage 218 through second coupling member 192. Cylindrical body portion 212 also includes a tube member 220 that extends from the outer surface of second coupling member 192 and that provide assess to main passage 218. The outer surface of second end 216 of second coupling member 192 provides a surface to which a delivery conduit may be sealingly attached.

In the example, rotary coupling device 60-3 is assembled by placing o-rings 222A and 222B into grooves 213A and 213B and inserting second end 200 of first coupling member 190 into first end 214 of second coupling member 192 in a manner such that port 208 is generally aligned with tube member 220. By doing so, secondary passage 204 will be in sealed fluid communication with tube member 220.

When assembled as just described, rotary coupling device 60-3 will include the two separate channels described elsewhere herein. In particular, main passage 202 of first coupling member 190 and main passage 218 of second coupling member 192 will together form the main channel of rotary coupling device 60-3 (e.g., for communicating breathing gas as described herein), and secondary passage 204 and tube member 220 will together form the secondary channel of rotary coupling device 60-3 (e.g., for providing pressure feedback as described herein).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim, "enumerating several" means, several of these means may be embodied by one and the same item of hardware The mere fact that certain elements are recited in mutually different dependent claims does not indicate that these elements cannot be used in combination.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A system (10, 150, 160, 170) for delivering a flow of breathing gas to a patient, comprising:
a main delivery conduit (22) coupled to a source of the flow of the breathing gas;
a rotary coupling device (60-2) coupled to the main delivery conduit (22), the rotary coupling device (60-2) having a first member (102) and a second member (104), the first member (102) defining a first channel (118) through the first member (102) and a second channel through the first member (102), the second member (104) defining a third channel (130) through the second member (104) and a fourth channel through the second member (104), wherein the first channel (118) is in communication with the third channel (130) to define a main channel having a main path through the rotary coupling device (60-2) that is in communication with the main delivery conduit (22), wherein the second channel is in communication with the fourth channel to define a secondary channel separate from the main channel and having a secondary path through the rotary coupling device (60-2), and wherein the first member (102) and the second member (104) are structured to freely rotate relative to one another for unlimited, 360° rotation about a longitudinal axis of the rotary coupling device (60-2) in a manner that separately maintains the integrity of the main path and the secondary path,
wherein the first member (102) comprises a first cylindrical portion (106) and a second, wider cylindrical portion (108) coupled to and extending from the first cylindrical portion (106), the first cylindrical portion (106) comprises a two-walled structure including an outer cylindrical wall (110) and including a first inner cylindrical wall (112) so that the outer surface of inner cylindrical wall (112) provides a surface to which the delivery conduit (22) is sealingly attached, and the second cylindrical portion (108) including a first outer cylindrical wall (114) and a second inner cylindrical wall (116), wherein the first member (102) further comprises a first port (120) that provides access to a space between the first outer cylindrical wall (114) and the second inner cylindrical wall (116), wherein the first and second inner cylindrical walls (112, 116) define the first channel (118) and the first port (120) comprises part of the second channel; and
wherein the second member (104) comprises a third cylindrical portion (122) and a fourth, narrower cylindrical portion (124) coupled to and extending from the third cylindrical portion (122), the third cylindrical portion (122) including a second outer cylindrical wall (126) and a third inner cylindrical wall (128), wherein the second member (104) further comprises a second port (132) that provides access to a space between the second outer cylindrical wall (126) and the third inner cylindrical wall (128), wherein the third inner cylindrical wall (128) and the fourth cylindrical portion (124) together define the third channel and the second port (132) comprises part of the fourth channel,
wherein a chamber (134) is formed between the second cylindrical portion (108) of the first coupling member (102) and the third cylindrical portion (122) of the second coupling member (104), wherein the chamber (134), the first port (120) and the second port (132) together form the secondary channel.

2. The system according to claim 1, wherein the system is a pressure support system adapted to provide a regimen of pressure support therapy to a patient, wherein the source is a pressure generating device (12), wherein the system further includes a patient interface device (40), and wherein the secondary channel is coupled to a port (5) of the patient interface device for providing pressure feedback from the patient interface device.

3. The system according to claim 2, wherein the secondary channel is coupled to a pressure sensor (14) within the pressure generating device.

4. The system according to claim 2, wherein the secondary channel is coupled to a sensor module (152) external to and in communication with the pressure generating device.

5. The system according to claim 1, wherein the system is a pressure support system adapted to provide a regimen of respiratory therapy to a patient, wherein the source is a pressure generating device (12), wherein the system further includes a patient interface device (40) and a source of supplemental gas (172), and wherein the secondary channel is coupled to both the source of supplemental gas (172) and a the patient interface device for providing a flow of the supplemental gas to the patient interface device.

6. The system according to claim 5, wherein the source of supplemental gas provides oxygen.

## Patentansprüche

1. System (10, 150, 160, 170) zur Zufuhr eines Beatmungsgasstroms an einen Patienten, umfassend:
eine Hauptzufuhrleitung (22), die mit einer Quelle des Beatmungsgasstroms gekoppelt ist;
eine Rotationskopplungsvorrichtung (60-2), die an die Hauptzufuhrleitung (22) gekoppelt ist, wobei die Rotationskopplungsvorrichtung (60-2) ein erstes Element (102) und ein zweites Element (104) aufweist, wobei das erste Element (102) einen ersten Kanal (118) durch das erste Element (102) hindurch und einen zweiten Kanal durch das erste Element (102) hindurch definiert, wobei das zweite Element (104) einen dritten Kanal (130) durch das zweite Element (104) hindurch und einen vierten Kanal durch das zweite Element (104) hindurch definiert, wobei der erste Kanal (118) in Kommunikation mit dem dritten Kanal (130) steht, um einen Hautkanal zu definieren, der einen Hauptweg durch die Rotationskopplungsvorrichtung (60-2) hindurch aufweist, die in Kommunikation mit der Hauptzufuhrleitung (22) steht, wobei der zweite Kanal in Kommunikation mit dem vierten Kanal steht, um einen sekundären Kanal zu definieren, der separat von dem Hauptkanal ist und einen sekundären Weg durch die Rotationskopplungsvorrichtung (60-2) aufweist, und wobei das erste Element (102) und das zweite Element (104) derart strukturiert sind, um sich relativ zueinander für eine unbegrenzte 360° Rotation um eine Längsachse der Rotationskopplungsvorrichtung (60-2) in einer Weise frei zu rotieren, in welcher die Integrität des Hauptwegs und des sekundären Wegs aufrechterhalten ist,
wobei das erste Element (102) einen ersten zylindrischen Teil (106) und einen zweiten, breiteren zylindrischen Teil (108) umfasst, der an den ersten zylindrischen Teil (106) gekoppelt ist und sich von diesem erstreckt, wobei der erste zylindrische Teil (106) eine doppelwandige Struktur umfasst, die eine äußere zylindrische Wand (110) beinhaltet, und
die eine erste innere zylindrische Wand (112) beinhaltet, sodass die Außenfläche der inneren zylindrischen Wand (112) eine Fläche bereitstellt, an welcher die Zufuhrleitung (22) auf dichtende Weise befestigt ist,
und wobei der zweite zylindrische Teil (108) eine erste äußere zylindrische Wand (114) und eine zweite innere zylindrische Wand (116) beinhaltet, wobei das erste Element (102) ferner eine erste Öffnung (120) umfasst, die Zugang zu einem Raum zwischen der ersten äußeren zylindrischen Wand (114) und der zweiten inneren zylindrischen Wand (116) bereitstellt, wobei die ersten und zweiten inneren zylindrischen Wände (112, 116) den ersten Kanal (118) definieren, und die erste Öffnung (120) Teil des zweiten Kanals umfasst; und
wobei das zweite Element (104) einen dritten zylindrischen Teil (122) und einen vierten, engeren zylindrischen Teil (124) umfasst, der an dem dritten zylindrischen Teil (122) gekoppelt ist und sich von diesem erstreckt, wobei der dritte zylindrische Teil (122) eine zweite äußere zylindrische Wand (126) und eine dritte innere zylindrische Wand (128) beinhaltet, wobei das zweite Element (104) ferner eine zweite Öffnung (132) umfasst, die Zugang zu einem Raum zwischen der zweiten äußeren zylindrischen Wand (126) und der dritten inneren zylindrischen Wand (128) bereitstellt, wobei die dritte innere zylindrische Wand (128) und der vierte zylindrische Teil (124) zusammen den dritten Kanal definieren, und die zweite Öffnung (132) Teil des vierten Kanals umfasst,
wobei eine Kammer (134) zwischen dem zweiten zylindrischen Teil (108) des ersten Kopplungselements (102) und dem dritten zylindrischen Teil (122) des zweiten Kopplungselements (104) gebildet ist, wobei die Kammer (134), die erste Öffnung (120) und die zweite Öffnung (132) zusammen den sekundären Kanal bilden.

2. System nach Anspruch 1, wobei das System ein Stützdrucksystem ist, das derart angepasst ist, um eine Behandlungsweise einer Stützdrucktherapie an einen Patienten bereitzustellen, wobei die Quelle eine Druckerzeugungsvorrichtung (12) ist, wobei das System ferner eine Patientenschnittstellenvorrichtung (40) beinhaltet und wobei der sekundäre Kanal an eine Öffnung (5) der Patientenschnittstellenvorrichtung zum Bereitstellen von Druck-Feedback von der Patientenschnittstellenvorrichtung gekoppelt ist.

3. System nach Anspruch 2, wobei der sekundäre Kanal an einen Drucksensor (14) innerhalb der Druckerzeugungsvorrichtung gekoppelt ist.

4. System nach Anspruch 2, wobei der sekundäre Kanal an ein Sensormodul (152) extern der Druckerzeugungsvorrichtung gekoppelt ist und mit dieser in Kommunikation steht.

5. System nach Anspruch 1, wobei das System ein Stützdrucksystem ist, das derart angepasst ist, um eine Behandlungsweise einer Stützdrucktherapie an einen Patienten bereitzustellen, wobei die Quelle eine Druckerzeugungsvorrichtung (12) ist, wobei das System ferner eine Patientenschnittstellenvorrichtung (40) und eine Quelle von zusätzlichem Gas (172) beinhaltet und wobei der sekundäre Kanal sowohl an die Quelle des zusätzlichen Gases (172) als auch an der Patientenschnittstellenvorrichtung zum Bereitstellen eines zusätzlichen Gasstroms an die Patientenschnittstellenvorrichtung gekoppelt ist.

6. System nach Anspruch 5, wobei die Quelle des zusätzlichen Gases Sauerstoff bereitstellt.

## Revendications

1. Système (10, 150, 160, 170) pour distribuer un flux de gaz respiratoire à un patient, comprenant :
une conduite de distribution principale (22) couplée à une source du flux de gaz respiratoire ;
un dispositif de couplage rotatif (60-2) couplé à la conduite de distribution principale (22), le dispositif de couplage rotatif (60-2) comprenant un premier élément (102) et un second élément (104), le premier élément (102) définissant un premier canal (118) à travers le premier élément (102) et un deuxième canal à travers le premier élément (102), le second élément (104) définissant un troisième canal (130) à travers le second élément (104) et un quatrième canal à travers le second élément (104), dans lequel le premier canal (118) est en communication avec le troisième canal (130) pour définir un canal principal comprenant un trajet principal à travers le dispositif de couplage rotatif (60-2) qui est en communication avec la conduite de distribution principale (22), dans lequel le deuxième canal est en communication avec le quatrième canal pour définir un canal secondaire distinct du canal principal et comprenant un trajet secondaire à travers le dispositif de couplage rotatif (60-2), et dans lequel le premier élément (102) et le second élément (104) sont structurés pour tourner librement l'un par rapport à l'autre pour une rotation à 360° illimitée autour d'un axe longitudinal du dispositif de couplage rotatif (60-2) d'une manière qui maintient séparément l'intégrité du trajet principal et du trajet secondaire,
dans lequel le premier élément (102) comprend une première partie cylindrique (106) et une deuxième partie cylindrique plus large (108) couplée à et s'étendant à partir de la première partie cylindrique (106), la première partie cylindrique (106) comprend une structure à double paroi comprenant une paroi cylindrique externe (110) et
comprenant une première paroi cylindrique interne (112) de sorte que la surface externe de paroi cylindrique interne (112) fournisse une surface à laquelle la conduite de distribution (22) est fixée de manière étanche,
et la deuxième partie cylindrique (108) comprenant une première paroi cylindrique externe (114) et une deuxième paroi cylindrique interne (116), dans lequel le premier élément (102) comprend en outre un premier orifice (120) qui fournit un accès à un espace entre la première paroi cylindrique externe (114) et la deuxième paroi cylindrique interne (116), dans lequel les première et deuxième parois cylindriques internes (112, 116) définissent le premier canal (118) et le premier orifice (120) comprend une partie du deuxième canal ; et
dans lequel le second élément (104) comprend une troisième partie cylindrique (122) et une quatrième partie cylindrique plus étroite (124) couplée à et s'étendant à partir de la troisième partie cylindrique (122), la troisième partie cylindrique (122) comprenant une seconde paroi cylindrique externe (126) et une troisième paroi cylindrique interne (128), dans lequel le second élément (104) comprend en outre un second orifice (132) qui fournit un accès à un espace entre la seconde paroi cylindrique externe (126) et la troisième paroi cylindrique interne (128), dans lequel la troisième paroi cylindrique interne (128) et la quatrième partie cylindrique (124) définissent ensemble le troisième canal et le second orifice (132) comprend une partie du quatrième canal,
dans lequel une chambre (134) est formée entre la deuxième partie cylindrique (108) du premier élément de couplage (102) et la troisième partie cylindrique (122) du second élément de couplage (104), dans lequel la chambre (134), le premier orifice (120) et le second orifice (132) forment ensemble le canal secondaire.

2. Système selon la revendication 1, dans lequel le système est un système d'assistance par pression adapté à fournir un régime de thérapie d'assistance par pression à un patient, dans lequel la source est un dispositif de génération de pression (12), dans lequel le système comprend en outre un dispositif d'interface patient (40), et dans lequel le canal secondaire est couplé à un orifice (5) du dispositif d'interface patient pour fournir un retour d'informations de pression à partir du dispositif d'interface patient.

3. Système selon la revendication 2, dans lequel le canal secondaire est couplé à un capteur de pression (14) à l'intérieur du dispositif de génération de pression.

4. Système selon la revendication 2, dans lequel le canal secondaire est couplé à un module de capteur (152) externe au et en communication avec le dispositif de génération de pression.

5. Système selon la revendication 1, dans lequel le système est un système d'assistance par pression adapté à fournir un régime de thérapie respiratoire à un patient, dans lequel la source est un dispositif de génération de pression (12), dans lequel le système comprend en outre un dispositif d'interface patient (40) et une source de gaz supplémentaire (172), et dans lequel le canal secondaire est couplé à la fois à la source de gaz supplémentaire (172) et au dispositif d'interface patient pour fournir un flux du gaz supplémentaire au dispositif d'interface patient.

6. Système selon la revendication 5, dans lequel la source de gaz supplémentaire fournit de l'oxygène.
